Europäisches Patentamt

European Patent Office

Office européen des brevets

(19)

(11) **EP 0 485 772 B1**

(12) **EUROPÄISCHE PATENTSCHRIFT**

| | |
|---|---|
| (45) Veröffentlichungstag und Bekanntmachung des Hinweises auf die Patenterteilung: **30.12.1998  Patentblatt 1998/53** | (51) Int Cl.⁶: **G01N 15/08**, B02C 4/32, B02C 25/00, G01N 15/02 |

(21) Anmeldenummer: **91118099.0**

(22) Anmeldetag: **24.10.1991**

(54) **Mahlwerk**

Grinding mill

Appareil de mouture

(84) Benannte Vertragsstaaten:
**AT BE CH DE DK ES FR GB GR IT LI LU NL SE**

(30) Priorität: **13.11.1990  DE 4036066**

(43) Veröffentlichungstag der Anmeldung:
**20.05.1992  Patentblatt 1992/21**

(60) Teilanmeldung: **98202206.3**

(73) Patentinhaber: **BUEHLER AG**
**9240 Uzwil (CH)**

(72) Erfinder:
- **Graenicher, Peter**
  **CH-9230 Flawil (CH)**

- **Braeker, Willy**
  **CH-9500 Wil (CH)**
- **Gemsjaeger, Helmut**
  **W-3300 Braunschweig (DE)**

(74) Vertreter: **Frommhold, Joachim, Dr.**
**Bühler AG**
**Patentabteilung**
**9240 Uzwil (CH)**

(56) Entgegenhaltungen:
| | |
|---|---|
| DE-A- 1 598 471 | DE-A- 3 519 625 |
| DE-A- 3 623 833 | DE-B- 1 129 735 |
| GB-A- 1 166 332 | US-A- 2 392 636 |

**Beschreibung**

Die Erfindung bezieht sich auf eine Vorrichtung nach dem Oberbegriffe des Anspruches 1.

Bei Walzwerken zum Mahlen von Schüttgütern ist es bekannt, den Walzenspalt zu regeln. Dieser Walzenspalt bestimmt u.a. die Maximalkorngrösse des Schüttgutes, das zwischen zwei Walzen hindurchgelassen wird. Es versteht sich, dass aber unterhalb dieser Maximalgrösse im Prinzip die verschiedensten Partikelgrössen möglich sind, wenngleich im allgemeinen ein relativ schmales Korngrössenspektrum angestrebt wird. Eine derartige Regelung eines Walzwerkes ist beispielsweise in der US-PS 3 468 488 beschrieben.

Ebenso ist es aus der US-PS 3 716 196 bekannt, eine Schlagmühle dadurch zu regeln, dass das Produkt anschliessend einer einfachen Siebung mit anschliessender Bestimmung des Anteiles der Ubergrössen unterzogen wird. Auch hier wird also durch die Lochgrösse des Siebes eine Maximalkorngrösse vorgegeben, wogegen die geringeren Partikelgrössen für die Regelung unberücksichtigt bleiben.

Schliesslich sei noch die CH-PS 438 760 erwähnt, aus der es bekannt ist, die Grösse von Spalten (nicht aber von festen Körpern) auf pneumatischem Wege zu messen.

Selbst wenn man zur Erkenntnis käme, dass auch die Bestimmung der kleineren Partikelgrössen für viele Vorgänge wichtig sind, so wird dies im allgemeinen durch mehrfache Siebung ermittelt, was ein zeitraubender Vorgang ist.

Eine solche mehrfache Siebung nach einer Walzenmühle ist beispielsweise aus der DE-A-35 19 625 bekannt geworden. Am Ausgange des Mahlwerkes wird das Mahlgut zunächst einmal durch eine Reihe von Sieben in verschiedene Fraktionen aufgeteilt. Aus dem Anteil einer Fraktion an der Gesamtmenge wird sodann geschlossen, ob der Walzenabstand den gesetzten Sollbedingungen entspricht oder verändert werden muss. Bereits die Beschreibung dieses Dokuments deutet an, dass ein solches System an sich der Gefahr ausgesetzt ist, "ins Schwingen" zu geraten. Dies rührt daher, dass ein Mahlvorgang relativ rasch abläuft, dass aber die Zeit bis das Produkt gesiebt und die entsprechende Fraktion dem Probennehmer zur Durchführung von Wiegemessungen zugeführt wurde ziemlich lang ist. Das aber ist wiederum durch die Art der Messung bedingt, die ja nur den Anteil einer Fraktion feststellt. Dieser Anteil kann Shwankungen unterworfen sein, die nicht allein auf den Mahlspalt zurückzuführen sind.

Zwar ist es, meist in der Zementindustrie, auch schon bekannt Proben des Produktes ins Laboratorium zur Durchführung von Korngrössenmessungen zu bringen (z.B. US-A-2 392 636). Dies erfolgt beispielsweise in einem Proberohr, das mit dem Produkt gefüllt und dessen pneumatische Durchlässigkeit geprüft wird. Vorgeschlagen wurde auch, eine solche Vorrichtung im Anschluss an eine Siebung in einen Regelkreis einzubinden, bei dem letztlich das Sieb beeinflußt wird (siehe GB-A-1 166 332).

Der Erfindung liegt die Aufgabe zugrunde, ein rasch arbeitendes Verfahren zur Regelung eines Mahlwalzwerkes heranzuziehen. Erfindungsgemäss weist eine derartige Einrichtung die im kennzeichnenden Teil des Anspruchs 1 angeführten Merkmale auf.

Weitergehende Einzelheiten der Erfindung werden in den Unteransprüchen beschrieben, welche hiermit als integrierender Bestandteil in die Beschreibung übernommen gelten sollen.

Permeabilitätsmessgeräte, wie sie für die Zwecke der Erfindung Anwendung finden sollen, sind z.B. in DIN 66126 beschrieben, wobei es sich um das Messprinzip nach Lea und Nurse oder nach Blaine handelt. Gemäss der genannten DIN-Vorschrift kann damit die spezifische Oberfläche pulverförmiger Stoffe gemessen werden. Der Erfindung liegt nun die Erkenntnis zugrunde, dass die spezifische Oberfläche ja in einem bestimmten Verhältnis zur Partikelgrösse stehen müsste, und dass es deshalb möglich sein müsste, mit Hilfe solcher bekannter Geräte die Mahlfeinheit einer Probe des Schüttgutes zu ermitteln. Dies kann beispielsweise so geschehen, dass gleichzeitig mit der zu messenden Probe eine Referenzprobe gemessen wird, von der die durchschnittliche Partikelgrösse bekannt ist, sodass hier ein einfacher Schluss auf die Partikelgrösse der zu messenden Probe möglich ist. Bevorzugt wird das Verfahren jedoch so durchgeführt, dass zunächst eine Vergleichsmessung von durchschnittlicher Partikelgrösse und Strömungswiderstand einer Probe durchgeführt wird, worauf aus dieser Vergleichsmessung eine Korrelationsrechnung durchgeführt wird, welche die Beziehung zwischen Durchschnittspartikelgrösse und Strömungswiderstand herstellt, und dass schliesslich bei wenigstens einer weiteren Probe lediglich der Strömungswiderstand gemessen und sein Wert mit der aus der Korrelationsrechnung ermittelten Regressionsformel gewonnen wird.

Es hat sich nun in einer Versuchsreihe erwiesen, dass die Zusammenhänge weitgehend als linear betrachtet werden können und deshalb eine lineare Regression der Form

$$y = m \cdot x + b$$

zur Errechnung der Partikelgrösse genügt. Im Prinzip braucht dabei die erste Referenzmessung zur Gewinnung des Faktors m und eines produktspezifischen Additionswertes b nur ein einziges Mal durchgeführt werden, worauf dann diese Werte für weitere Messungen an vergleichbarem Schüttgut für alle Zukunft zur Verfügung stehen. Insoferne braucht also dieser erstgenannte Schritt nicht immer durchgeführt werden.

Es wurde bereits erwähnt, dass der Mahlspalt einer Mühle und insbesondere eines Walzwerkes mit wenigstens zwei relativ zueinander bewegbaren Mahlteilen nur die Maximalpartikelgrösse, nicht aber die Durchschnittspartikelgrösse bestimmt. Dennoch kann man mit einem entsprechenden Verfahren, wie oben beschrieben, auch den Mahlspalt bestimmen, denn es bedarf hierzu bloss einer Referenz-oder Eichmessung, um einen Faktor m zu gewinnen, bei dessen Multiplikation mit dem jeweiligen Strömungswiderstand bzw. Addition des Werts b bei der Messung einer anderen Probe die Maximalpartikelgrösse und damit indirekt der benötigte Mahlspalt ermittelt werden kann.

Dies kann nun erfindungsgemäss bei einer Mühle der genannten Art dazu eingesetzt werden, dass im Anschluss an diese Mühle ein Probenehmer vorgesehen ist, dem ein Permeabilitätsmessgerät nachgeschaltet ist, dessen dem Strömungswiderstand entsprechender Messwert einer Regeleinrichtung für die Mühle zugeführt wird.

Bei Gutbettzerkleinerung ist es bekannt, dass zur Regelung der Mühle verschiedene Einrichtungen und Parameter herangezogen werden können, wie Geschwindigkeit der Zufuhreinrichtung, Geschwindigkeit der Walzen oder Einstellung des Mahldruckes. Bevorzugt wird jedoch über die Regeleinrichtung eine Abstandsregeleinrichtung für die zueinander bewegbaren Mahlteile, wie insbesondere Mahlwalzen, zur Einstellung der Spaltgrösse geregelt.

Weitere Einzelheiten ergeben sich anhand der nachfolgenden Beschreibung eines in der Zeichnung schematisch dargestellten Ausführungsbeispieles. Darin zeigen:

Fig. 1        eine perspektivische Darstellung einer mit einer erfindungsgemässen Messeinrichtung zur Regelung ausgerüsteten Mühle, von der

Fig. 2        einen Schnitt nach der Linie II-II der Fig. 1 und

Fig. 3        die Messeinrichtung im Detail im Schnitt nach der Linie III-III der Fig. 1 veranschaulicht, wogegen an Hand der

Fig. 4a bis 4c        drei Varianten zur Regelung der Schüttdichte erläutert werden, wobei Fig. 4a einen vergrösserten Schnitt durch die Kompaktiereinrichtung, ähnlich der Schnittführung der Fig. 2, darstellt und die Fig. 4b und 4c zwei Ausführungen einer Klappenbetätigung am Auslauf der Kompaktiereinrichtung in einem teilweisen Längsschnitt zeigen; und

Fig. 5        ein Diagramm zur Veranschaulichung der Korngrössenverteilungen der Beispiele nach der beschriebenen Tabelle.

Fig. 6        zeigt die zugehörige Partikelgrössenverteilung.

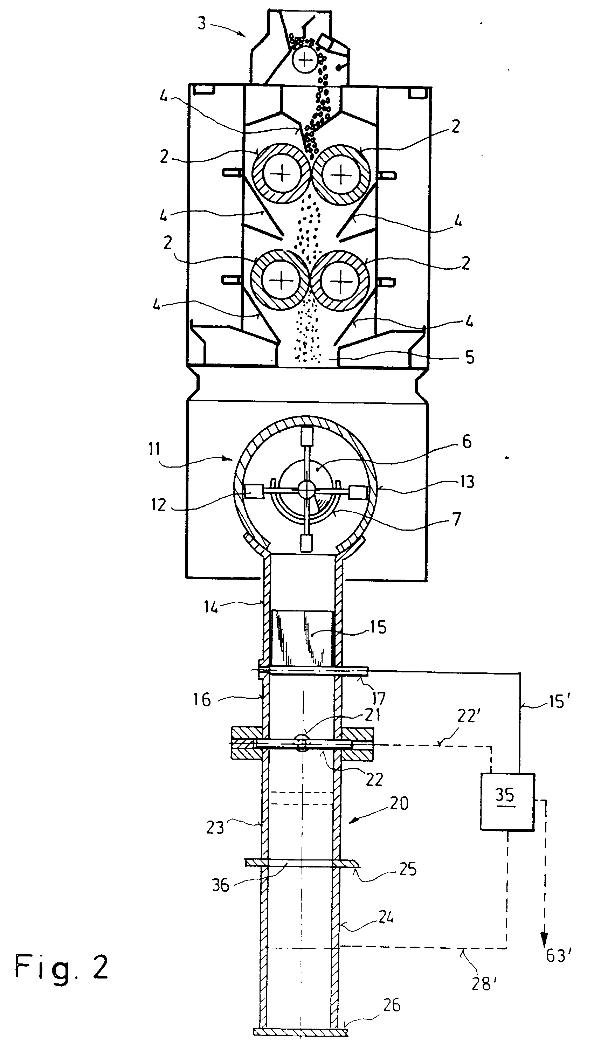

Eine Walzenmühle 1 ist zum Trockenvermahlen von Schüttgut und insbesondere von relativ sprödem oder versprödetem Gut, z.B. von gerösteten Nahrungsmitteln, wie Kaffee, ausgebildet. Sie besitzt zu diesem Zwecke wenigstens ein Paar, vorzugsweise wenigstens zwei Paare von Walzen 2. Wie Fig. 2 zeigt, wird diesen Walzen 2 Schüttgut über eine Speiseeinrichtung 3 direkt in den Walzenspalt des ersten Paares von Walzen 2 zugeführt, die das Schüttgut vorvermahlen, und anschliessend einem zweiten Walzenpaar 2, wobei Zufuhrflächen 4 dafür sorgen, dass das trockene Schüttgut in den jeweils darunterliegenden Walzenspalt bzw. in einen Auslauf 5 gelangen.

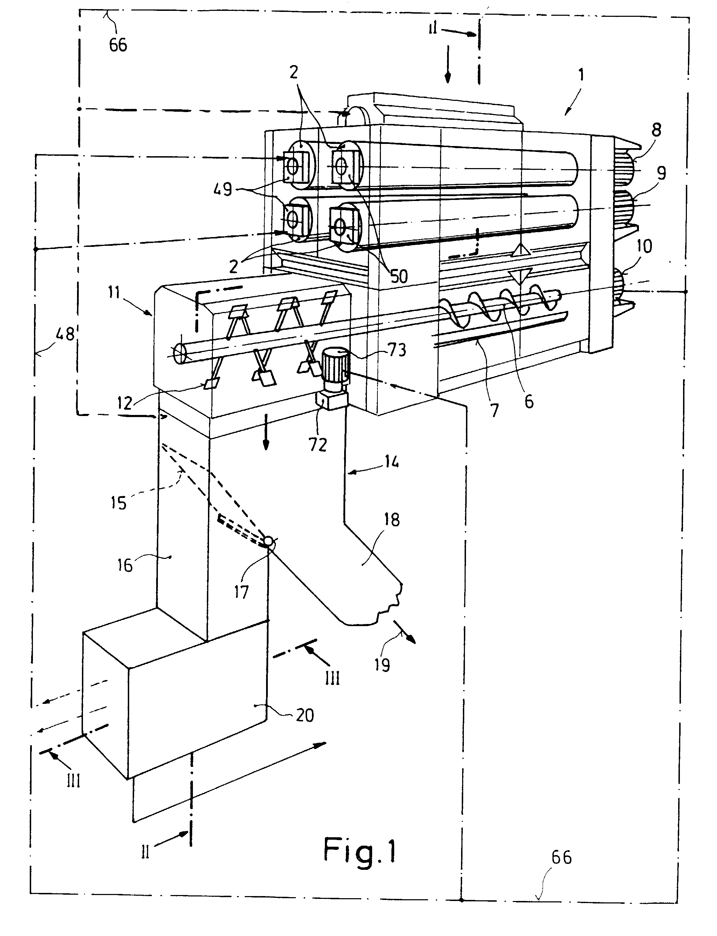

Unterhalb des Auslaufes 5 ist eine, am besten aus Fig. 1 erkennbare Sammelschnecke 6 in einem rinnenförmigen Schneckengehäuse 7 angeordnet. Während den Walzenpaaren 2 jeweils eigene Antriebe 8 und 9 zugeordnet sind, besitzt die Sammelschnecke 6 einen gesonderten Motor 10, der über ein (nicht dargestelltes) Reduziergetriebe die Schnecke 6 antreibt.

Wie ferner aus Fig. 1 ersichtlich ist, ist gleichachsig mit der Schnecke 6 ein Homogenisier- und Kompaktierwerk 11 in einem eigenen Gehäuse angeordnet, das zur Durchmischung und Schüttdichtenveränderung mit Paddelarmen 12 versehen ist, die etwa radial abstehen. Dieses Homogenisier- und Kompaktierwerk 11 dient der Austragung des gemahlenen Produktes, wobei es sich versteht, dass die Ausbildung unterhalb der Walzen 2 an sich beliebig sein könnte, obwohl gerade für die beabsichtigte Messung der Durchschnittsmahlfeinheit, aber auch zu einer erwünschten Schüttdichtenbestimmung die Durchmischung und Schüttdichtenveränderung von besonderem Vorteil ist. Es sei jedoch erwähnt, dass an Stelle der gezeigten Werkzeuge, beispielsweise für den Anwendungsfall im Anschluss an eine Gutbettzerkleinerung, auch Auflösungswerkzeuge zur Auflösung von bei der Gutbettzerkleinerung im allgemeinen anfallenden Agglomeraten vorgesehen sein können.

Wie besonders aus Fig. 2 ersichtlich ist, sitzt das Kompaktierwerk 11 in einem die Paddelarme 12 relativ eng umgebenden Gehäuse 13. An dieses Gehäuse 13 schliesst nach unten zu eine, ganz allgemein aus Fig. 1 ersichtliche Rohrleitung 14 oder von einer eckigen auf eine runde Querschnittsform überleitende Trimelle 14. Von dieser Rohrleitung 14 zweigt eine normalerweise durch eine Klappe 15 verschlossene Probenehmerleitung 16 ab. Die Klappe 15 (an ihrer Stelle kann auch ein anderer Probenehmer vorgesehen sein) ist um eine Achse 17 schwenkbar, die in zeitlichen Abständen über eine nicht dargestellte Betätigungseinrichtung derart schwenkbar ist, dass der Rohrleitungsabschnitt 16 freigegeben wird. Während also normalerweise das fertig gemahlene Gut über einen Rohrleitungsabschnitt 18 im Sinne des Pfeiles 19 an einen Silo, eine Absackeinrichtung oder einen Förderer abgegeben wird, fällt ein Teil des aus der Homogenisiereinrichtung 11 tretenden Gutes bei entsprechender Stellung der Klappe 15 in den Probenehmerschacht 16. Die Betätigung der Klappe 15 wird im allgemeinen über ein Zeitglied in regelmässigen Zeitabständen erfolgen, doch kann es für manche Anwendungsfälle bevorzugt sein, einen Zufallsgenerator einzusetzen, über den die Klappe 15 in unregelmässigen Zeitabständen betätigt wird.

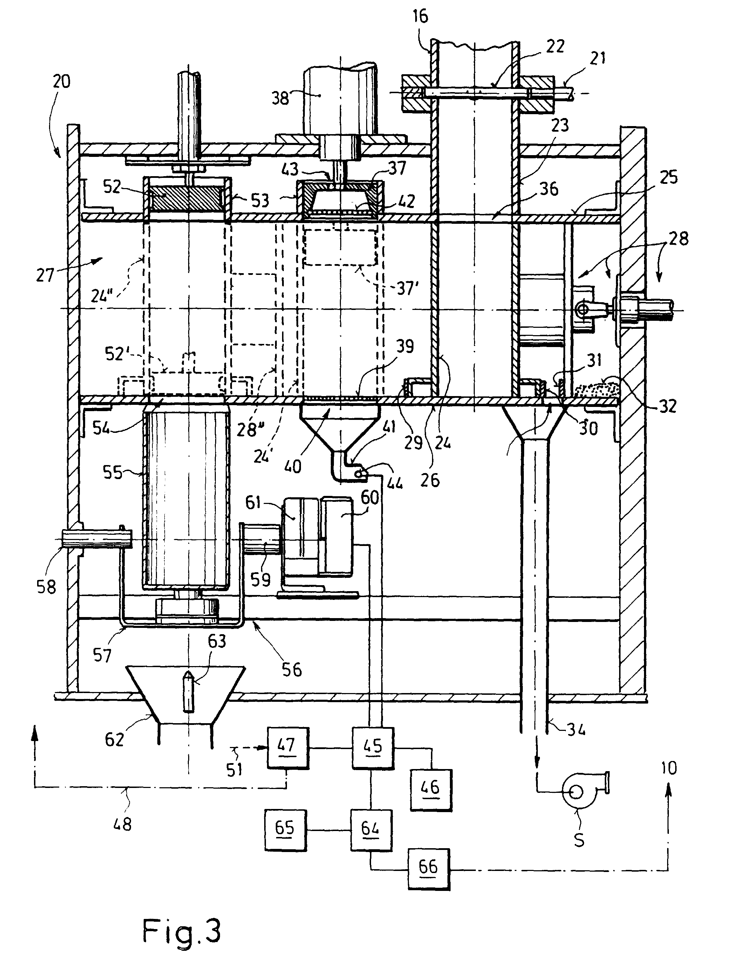

An den Probenehmerabschnitt 16 schliesst das Gehäuse einer Messeinrichtung 20, deren Einzelheiten den Fig. 2 und 3 zu entnehmen sind. Demnach ist die Messeinrichtung 20 an den Rohrleitungsabschnitt 16 angeflanscht, wobei dazwischen ein über eine Stange 21 betätigbarer Absperrschieber 22 vorgesehen sein kann. Wie später noch erläutert wird, wird durch diesen Absperrschieber 22 gesichert, dass das Probematerial stets aus einer vorbestimmten Fallhöhe über einen innerhalb des Gehäuses 20 angeordneten und an den Rohrabschnitt 16 angeflanschten Stutzen 23 in ein darunterliegendes Probengefäss 24 fällt.

Das Gefäss 24 ist innerhalb eines von einer Deckplatte 25 und einer Bodenplatte 26 begrenzten Schieberaumes 27 mit Hilfe eines, beispielsweise mittels eines nicht dargestellten fluidischen Antriebes oder über den Schneckenantrieb eines Motors antreibbaren Schieber 28 in drei verschiedene, strichliert dargestellte Lagen entlang des Bodens 26 verschiebbar.

Vorzugsweise besitzt das Probengefäss 24 an seiner dem Boden 26 zugekehrten Unterseite wenigstens eine Abstreifleiste 29 bzw. 30, durch welche allenfalls über schwer abzudichtende Oeffnungen auf die Oberseite des Bodens 26 gefallene Partikel des zu messenden Schüttgutes zur Seite gestreift werden, um Reibungsstörungen zu vermeiden. Auch der Schieber 28 kann an seiner dem Boden 26 zugewendeten Seite mit einer Abstreifleiste 31 versehen sein, obwohl es an sich auch möglich ist, die Schieberwandung oberhalb des Bodens 26 so weit enden zu lassen, dass sich über dem Boden eine Schlitzöffnung befindet.

Durch diese Abstreifleisten 29 bis 31 wird etwaiges Schüttgut zur Seite (und allenfalls zum Rohr 34) geschoben, wie aus angedeuteten Schüttguthäufchen 32 erkennbar ist. Bevorzugt ist es jedoch, innerhalb des Bodens 26 im Bereiche wenigstens einer Endstellung einer solchen Abstreifleiste (hier die Abstreifleiste 30) eine Offnung 33 vorzusehen, an die zumindest eine in das Gehäuse 20 mündende, z.B. zur Gutabfuhr 19 (Fig. 1) führende Leitung 34 und allenfalls an eine Absaugeinrichtung s angeschlossen ist. Damit kann die Notwendigkeit periodischer Reinigungen zum Entfernen der Häufchen 32 vermieden werden, indem die Rohrleitung 34 mit ihrer Mündung 33 im Boden 26 für eine Selbstreinigung sorgt. Es versteht sich, dass auch an jenen Stellen, in denen die Häufchen 32 angedeutet sind, der Leitung 34 entsprechende Reinigungsleitungen vorgesehen sein können.

Wie schon erwähnt, kann die Klappe 15 (Fig. 2) zeitabhängig gesteuert sein. Hierzu mag ein Programmsteuerwerk 35 vorgesehen sein, das die Bewegungen der einzelnen Teile über entsprechende Ausgänge 15', 22' und 28' zur Betätigung der Klappe 15, des Absperrschiebers 22 und des Schiebers 28 in der jeweils nötigen zeitlichen Aufeinanderfolge steuert. Auf diese Weise wird zunächst die Klappe 15 kurzzeitig geöffnet, um einen Teilstrom aus der Leitung 14 in die Probennehmerleitung 16 umzuleiten. Sobald die Klappe 15 die Zweigleitung 16 wieder verschlossen hat, wird der Absperrschieber 22 geöffnet, sodass die auf ihm ruhende Partikelmenge über den Rohrstutzen 23 in das Probengefäss 24 fällt. An sich mag es zweckmässig erscheinen, die Offnungszeit für die Klappe 15 so zu wählen, dass das Probegefäss 24 nicht überfüllt wird. Um es aber mit Sicherheit gänzlich zu füllen, wird diese Zeit vorzugsweise so bemessen, dass das Gefäss 24 stets überfüllt wird, wobei zum Wegräumen des Überschusses die Leisten 29 bis 31 (Fig. 3) vorgesehen sind. Es versteht sich ferner, dass in der Deckplatte 25 in Höhe des ortsfesten Stutzens 23 eine Offnung 36 vorgesehen ist.

Sobald dies geschehen ist, wird der Schieber 28 betätigt und verschiebt das Probegefäss 24 in die strichliert dargestellte Stellung 24'. An der der Lage 24' entsprechenden Station wird die eigentliche Messung im Sinne von DIN 66126 vorgenommen, d.h. es wird die Schüttgutprobe innerhalb des Probenbehälters 24 unter vorbestimmtem Drucke komprimiert. Zu diesem Zwecke ist an der Oberseite des Probegefässes 24 in seiner Stellung 24' ein Kolben 37 vorgesehen, der über ein Zylinder-Aggregat 38 in seine abgesenkte Stellung 37' unter dem vorbestimmten Drucke bewegbar ist, sodass die Schüttgutprobe unter diesem vorbestimmten Drucke in vorbestimmter Weise zusammengepresst wird.

Nach DIN 66126 muss sodann die Probe von einem Gas vorbestimmter Menge pro Zeiteinheit durchströmt werden, um die Permeabilität bzw. den Durchströmwiderstand zu messen (als Variante ist ein stets gleichbleibendes Gasvolumen denkbar). Im Prinzip wäre dies in einer von der Stellung 24' abweichenden, einem nächsten Bewegungsschritt entsprechenden Stellung möglich, doch kann das Messgerät 20 auch in der dargestellten Weise ausgeführt sein, wobei im Boden 26 eine durch ein Sieb 39 abgedeckte Offnung 40 vorgesehen ist. Das Sieb 39 hat eine Maschenweite, die klein genug ist, um dem Drucke des Kolbens 37 einen Widerstand entgegenzusetzen und die Partikel der Probe nicht hindurchzulassen.

An diese Offnung 40 ist nun eine Gaszufuhreinrichtung 41, insbesondere für Luftzufuhr, vorgesehen, um die im Gefässe 24 befindliche Probe zu durchströmen. Prinzipiell könnte die Anordnung auch so getroffen sein, dass anstelle des Siebes 39 ein Abdeckschieber vorgesehen ist, der dann geöffnet wird, wenn der Kolben 37 die Probe bereits zusammengepresst hat und in seine mit vollen Linien dargestellte Lage zurückgekehrt ist (was aber nicht unbedingt erfolgen muss), sodass durch Wegziehen dieses Schiebers die Oeffnung 40 für die Gaszufuhr freigegeben wird.

Der Kolben 37 seinerseits besitzt zweckmässig ebenfalls ein relativ engmaschiges Sieb 42, dessen Maschen klein genug sind, um einen wirksamen Druck auf die Probe ausüben zu können, aber gross genug, um das über die Zuleitung 41 herangebrachte und die Probe durchströmende Gas hindurchzulassen. An der Oberseite des Kolbens 37 mag dann wenigstens eine Oeffnung 43 vorgesehen sein, durch welche die zur Permeabilitätsmessung verwendete Luft ins Freie strömen kann oder über eine nicht dargestellte Leitung abgeleitet wird, beispielsweise um im Kreislauf wieder der Leitung 41 zugeführt zu werden. Wie schon erwähnt, ist es durchaus möglich, etwa das Sieb 39 oder eine andere Abdeckung (der erwähnte Schieber) an einer anderen Station vorzusehen, die dann selbstverständlich den Kolben 37 nicht aufzuweisen braucht. In diesem Falle mag dann der Kolben 37 ohne die Teile 42 und 43 ausgebildet sein. Innerhalb der Leitung 41 ist ein Sensor 44 an einer an sich beliebigen Stelle angeordnet, der den Druckwiderstand der im Gefäss 24 enthaltenen Probe misst und ein entsprechendes Ausgangssignal an einen Rechner 45 sendet, der von einem Geber 46 die jeweiligen Werte für m und b erhält und darauf entsprechende Signale an eine Regeleinrichtung 47 liefert. Dies kann von einem einzigen Geber 46 aus durch serielle Signale oder über parallele Leitungen erfolgen, oder es ist für die beiden Werte m und b jeweils ein gesonderter Geber vorgesehen.

Ferner errechnet der Rechner 45 aus dem bekannten Volumen des Probengefässes 24 und dem gemessenen Gewicht die Schüttdichte, die besonders dann wichtig ist, wenn das gemahlene Gut anschliessend in Verpackungen vorbestimmter Grösse und vorbestimmten Füllgewichtes eingefüllt werden soll. Auch hier wären Modifikationen denkbar, denn es wäre auch denkbar, dass das Schüttgut mit vorbestimmtem Druck durch den Kolben 37 zusammengepresst und dann an Hand der Kolbenstellung (bei Verwendung eines Kolbenpositionssensors) oder eines Niveausensors für das im Probengefäss 24 befindliche Schüttniveau (das hiezu beispielsweise durchsichtig ausgebildet ist, um eine optische Abtastung zu ermöglichen) das Volumen zu ermitteln.

Ein dem errechneten IST-Wert der Schüttdichte entsprechendes Signal wird also vom Rechner 45 einer Regelstufe 64 zugeführt, die einen Sollwert von einem einstellbaren Sollwertgeber 65 erhält. Daraus wird in der Regelstufe ein die Abweichung vom Soll-wert berücksichtigendes Regelsignal gewonnen, das einer Stellsignalstufe 66 zugeführt wird. Diese Stellsignalstufe 66 regelt die Drehgeschwindigkeit des Kompaktierwerkes 11 und damit dessen Kompaktierdruck (falls das Kompaktieren mit Hilfe anderer druckausübender Einrichtungen, wie Druckkolben od.dgl. erfolgt, wird deren Druck in analoger Weise geregelt).

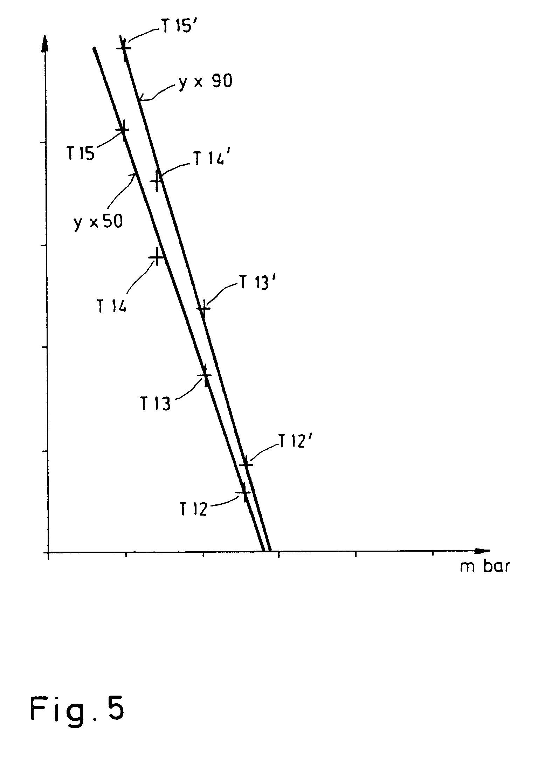

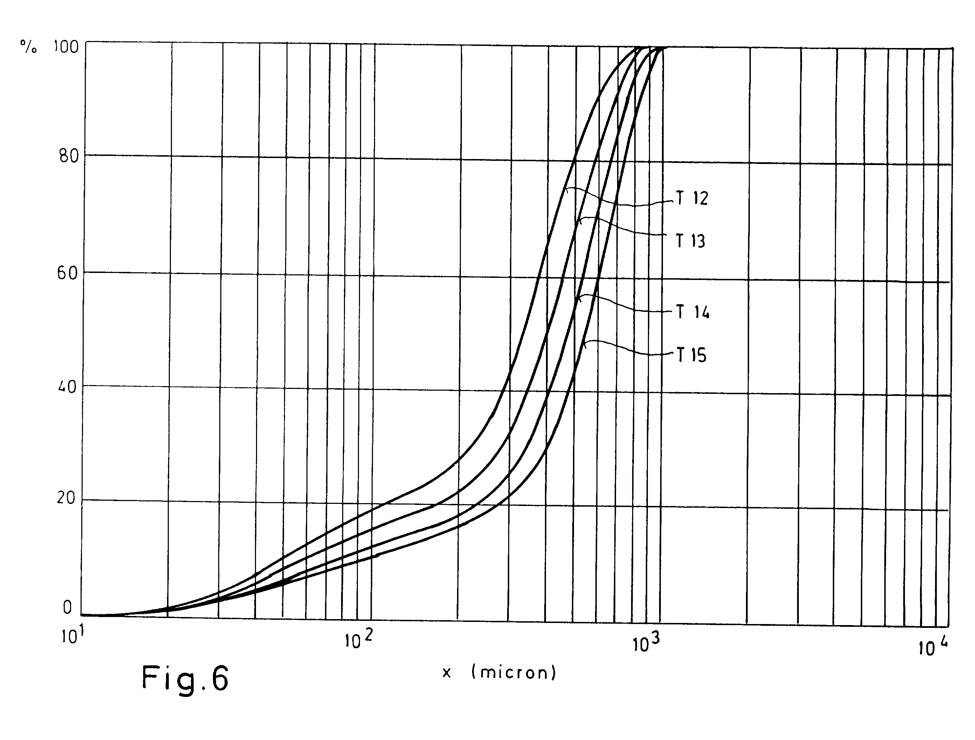

Der Geber 46 kann auf die verschiedenste Weise ausgebildet sein. Im Prinzip kann er nach Art der Station 24' konstruiert sein, um parallel zur Messung an der Stelle 24' eine Vergleichsmessung durchzuführen, wobei die Vergleichsmessung von einer Standardprobe bekannter Partikelgrösse gebildet ist. Falls aber die Eichung von Hand aus vorher vorgenommen wird, kann der Geber 46 aus einer einfachen Eingabetastatur für die betreffenden Werte von m und b für die Maximalpartikelgrösse (siehe die Erläuterungen zu Fig. 3 im Zusammenhang mit Fig 5 und 6) ausgebildet sein. Es versteht sich, dass, besonders bei Verwendung des Messgerätes 20 ohne die Mühle 1, über den Geber 46 dann dementsprechend die Werte für die durchschnittliche Partikelgrösse eingegeben werden, um diese zu erhalten. Dies kann im Falle einer Gutbettvermahlung ebenfalls von Vorteil sein. Eine weitere Möglichkeit besteht darin, dass der Geber 46 für die Eingabe von Daten eingerichtet ist, die die jeweiligen Werte für m und b erst bestimmen. Es sind dies etwa die Art des zu betrachtenden Produktes, wie etwa Röstkaffee, der durch die Röstbehandlung eine gewisse Sprödigkeit erreicht, oder anderes mehr oder minder sprödes Gut, wie Zement, Kohle oder Erz. Ferner könnten die Presskraft des Kolbens 37 (falls diese veränderbar ist) die Probenlänge (falls der Probenbehälter 24 nicht völlig gefüllt wird), der Druck durch die Leitung 41 (falls sich dieser ändern kann) und allenfalls auch die Schüttdichte (falls die Belastung des Kolbens 37 veränderbar ist) eingegeben werden.

Das Ausgangssignal der Rechenstufe 45, die praktisch eine blosse Multiplikationsstufe bekannter Ausbildung darstellt, wird der Regeleinrichtung 47 übermittelt, deren strichpunktierte Ausgangsleitung 48 auch in Fig. 1 zu sehen ist. Denn wenn das Rechenergebnis der Stufe 45 einem Soll-Wert für den Mahlspalt entspricht, so sind zweierlei Vorgangsweisen möglich: entweder es ist ein Positionssensor für die Grösse des Mahlspaltes, beispielsweise für die Relativlage beweglicher Walzenlager 49 gegenüber ortsfesten Walzenlagern 50 vorgesehen, dessen Ausgangssignal über eine strichliert in Fig. 3 gezeigte Leitung 51 als IST-Wert der Regeleinrichtung 47 zugeführt wird. Oder die Regeleinrichtung 47 arbeitet mit einem Inkrementalsystem (z.B. mit einem Schrittmotor) als (nicht dargestelltes) Stellglied, in welchem Falle ihr die jeweilige Stellung der beweglichen Lager 49 bekannt ist. Dies kann z.B. so erfolgen, dass mit dem Einschalten des Betriebes des Mahlwerkes 1 die Walzen 2 bis auf einen Mahlspalt von "0" zusammengefahren werden, um den Null-Punkt zu bestimmen, worauf sie in eine gewünschte und einstellbare Mahlspaltposition wieder auseinanderfahren. Dabei zählt die Regeleinrichtung 47 entweder die Schritte des Schrittmotors oder die Inkrementalschritte eines Inkremental-Weggebers. Da aber häufig die Mahlwalzen 2 eine empfindliche Oberfläche besitzen, ist es bevorzugt, wenn der umgekehrte Weg beschritten wird, indem die Walzen 2 zunächst auf eine definierte, z.B. durch einen Geber oder durch einen Anschlag bestimmte, Offenposition auseinanderfahren und dann erst in die gewünschte Mahlspaltposition zusammengebracht werden.

All diese Massnahmen sind in der Regeltechnik an sich bekannt und bedürfen daher keiner ins Einzelne gehenden Erläuterung. Vielmehr ist verständlich, dass auf diese Weise auch bei der Vermahlung von Trockengütern eine einfache Bestimmung der erzielten Durchschnittsmahlfeinheit und eine entsprechende Nachstellung eines Mühlenaggregates möglich ist. Aus der US-A-3 734 659 ist es beispielsweise bekannt, dass der Mahlspalt bzw. die Feinheit des Mahlgutes auch durch Regelung der Zufuhrmenge durch Beeinflussung der Speisung relativ zu einer gegebenen Umfangsgeschwindigkeit der Mahlwalzen beeinflusst werden kann. Dementsprechend ist auch die Regelung der Walzengeschwindigkeit bei gegebener Zufuhrgeschwindigkeit denkbar.

Wenn nun daher die im Probenbehälter 24 enthaltene Probe bereits in der Stellung 24' gemessen ist, könnte sie zur nächsten Stellung des Schiebers 28, der Stellung 28" bzw. der Stellung 24" des Probenbehälters 24 verschoben werden, wo ein Auswerferkolben 52 in einem ähnlichen Zylinderstutzen 53 geführt ist, wie der Kolben 37. Dieser Auswerferkolben durchfährt den gesamten Probenbehälter 24 und erreicht am Ende seiner Bewegung eine Stellung 52'.

In Fig. 3 ist nun aber noch eine Korrekturmöglichkeit vorgesehen und gezeigt. Wenn nämlich das gemessene Schüttgut aus dem Probenbehälter 24 in der Stellung 24" vom Auswerferkolben 52 durch eine Oeffnung 54 der Bodenplatte 26 gedrückt worden ist, kann es in einen Wiegebehälter 55 einer Waage 56 eingeschoben werden. Dieser Wiegebehälter 55 ist an einem etwa U-förmigen Bügel 57 befestigt, dessen Schenkel an Wellenstummeln 58, 59 sitzen. Von diesen Wellenstummeln 58, 59 ist wenigstens einer mit einer Druckmessdose 60 verbunden, über die das Gewicht der Probe bestimmbar ist. Obwohl bei völliger Füllung des Probenbehälters 24 das Gewicht stets gleich sein müsste, können sich geringere Differenzen ergeben, die als Korrekturwert aus der Messeinrichtung 60 in die Recheneinrichtung 45 eingegeben werden.

Es ist jedoch noch ein anderes Vorgehen denkbar, denn einerseits könnte auf den Kolben 37 verzichtet werden, wenn man davon ausgeht, dass die Fallhöhe beim Befüllen des Probenbehälters 24 dazu ausreicht, einen vorbestimmten Druck auf das Material auszuüben und damit eine vorbestimmte Schüttdichte zu erreichen. Zwar könnte für diese Fallhöhe die Länge des Rohrstutzens 23 samt der daran anschliessenden Probenehmerleitung 16 und der darüberliegenden Leitung 14 verwendet werden, doch ergeben sich dabei unter Umständen zuviele Störfaktoren, weshalb es vorteilhaft ist, den Absperrschieber 22 zur Definierung der Fallhöhe anzuordnen. Es könnte dann nach Durchströmung der so ohne den Kolben 37, lediglich aufgrund ihres Eigengewichtes verdichteten Probe mit Luft ein Wiegevorgang im Behälter 55 einsetzen, der allfällige Korrekturen liefert. Anderseits versteht es sich, dass der Wiegebehälter 55 samt Waage 56 auch völlig entfallen kann.

Sobald der Wiegevorgang beendet ist, wird der Wellenstummel 59 von einem mit der Gewichtsmesseinrichtung 60 verbundenen Antrieb 61 an sich beliebiger Art (Dreheiseninstrument, Motor, Elektromagnet, fluidischer Motor) gedreht, wobei der Behälter 55 in eine um 180° gedrehte Lage gelangt und sein Inhalt in einen, zweckmässig zur Einrichtung 19 (Fig. 1) führenden Trichter 62 entleert wird. Falls auf eine Waage 56 verzichtet wird, würde dieser Trichter 62 unmittelbar unter der Offnung 54 angeordnet sein. Es ist vorteilhaft - insbesondere um das Gewichtsmessergebnis nicht zu verfälschen - im Bereiche des Trichters 62 eine Reinigungseinrichtung für das Behälterinnere des Wiegebehälters 55 vorzusehen. Eine solche Reinigungseinrichtung kann im Prinzip aus einer rotierenden Bürste gebildet sein, doch ist es einfacher und zweckmässiger, hierfür wenigstens eine Fluiddüse 63 vorzusehen, insbesondere eine Druckluftdüse, deren Druckluftzufuhr beispielsweise durch das Programmwerk 35 (Fig. 2) über dessen Ausgangsleitung 63' gesteuert wird.

Es versteht sich, dass auch hinsichtlich der Ausbildung und Anordnung der Waage 56 manigfaltige Modifikationen denkbar sind, wie etwa die Unterbringung der Messdose 60 zwischen der Unterseite des Behälters 55 und dem Bügel 57, was lediglich den Nachteil besitzt, dass die Zuleitungen beweglich sein müssen. Ferner wurde das Messgerät 20 für eine lineare Verschiebung des Probenbehälters 24 mit Hilfe eines Schiebers 28 erläutert, welch letzterer selbstverständlich nach dem Auswerfen der Probe mittels des Kolbens 52 wieder in die mit vollen Linien dargestellte Lage zurückkehren kann; es wäre aber ebenso denkbar, die einzelnen Stationen 24, 24', 24" entlang einer Kreisbahn vorzusehen und in diesem Falle anstelle eines linear verschiebbaren Schiebers 28 eine Art Revolverkonstruktion vorzusehen, die um eine antreibbare Welle drehbar ist, zu der der Probenbehälter 24 exzentrisch angeordnet wird.

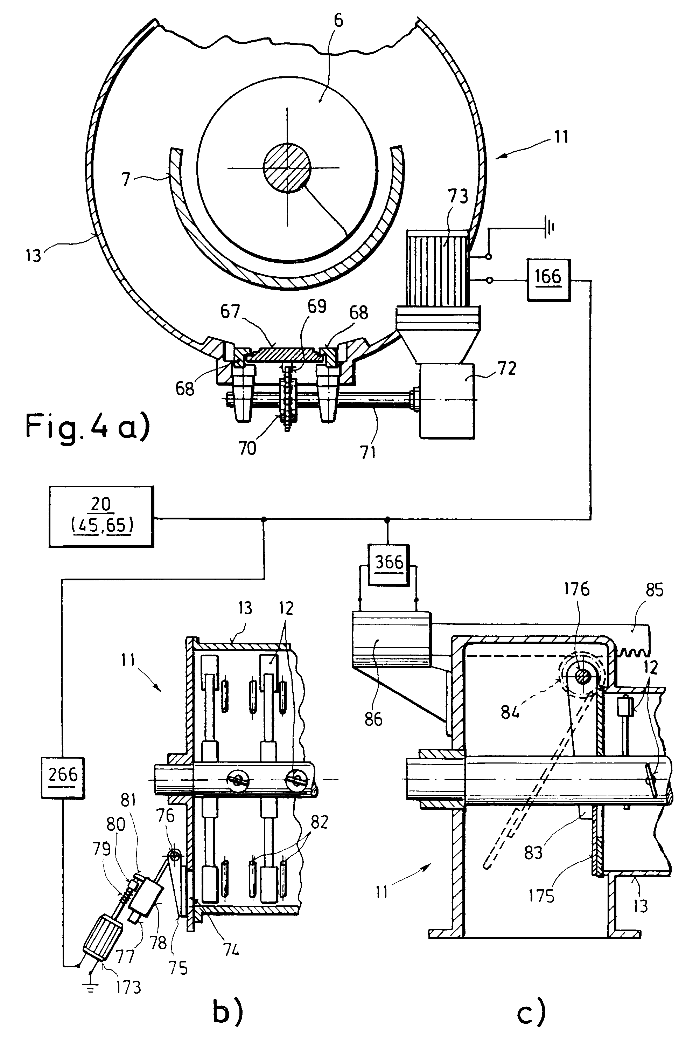

Fig. 4 zeigt in drei, durch die gemeinsame Messeinrichtung 20 bzw. 45, 64 verbundenen, Varianten a) bis c), wie das Ausgangssignal der Messeinrichtung 20 auch noch auf andere Weise verwendet werden kann. Nach Fig. 4 a) ist die Unterseite des Kompaktiergehäuses 13 durch einen Schieber 67 verschlossen, der in Führungen 68 verschiebbar geführt ist und den Auslauf des Kompaktierwerkes 11 mehr oder weniger verschliesst. Zu seiner Verschiebung ist der Schieber 67 an seiner Unterseite mit einer Kette 69 verbunden, die über ein Kettenrad 70 antreibbar ist. Im Prinzip könnte dieser Antrieb auch durch eine Zahnstange am Schieber 67 und ein darin eingreifendes Zahnrad (entsprechend dem Rad 70) erfolgen, doch ist der Kettenantrieb weniger empfindlich und daher bevorzugt.

Das Rad 70 sitzt auf einer Antriebswelle 71, die über ein Getriebe 72 ihren Drehantrieb von einem Stellmotor 73 (vgl. auch Fig. 1) erhält. Es ist aus Genauigkeitsgründen bevorzugt, wenn als Stellmotor 73 ein Schrittmotor verwendet wird. Dieser Schrittmotor 73 wird nun über eine Stellstufe 166 angesteuert, die funktionell der schon beschriebenen Stellstufe 66 entspricht und ihr Regelsignal von den Einrichtungen 45 und 64 erhält.

Im Falle der Fig. 4 b) hingegen ist die Einrichtung 20 mit einer Stellstufe 266 verbunden. Das Kompaktiergehäuse 13 besitzt hier nur eine relativ geringe Auslassöffnung 74, die durch eine um eine Achse 76 schwenkbare Klappe 75 als Gegendruckglied verschlossen ist. Diese Klappe 75 steht unter dem Drucke eines entlang eines Hebels 77 verstellbaren Gewichtes 78. Die Stellung dieses Gewichtes 78, und damit sein Andruckmoment auf die Klappe 75, wird durch die Stellung eines auf einer Gewindespindel verstellbaren Anschlages 80 bestimmt, der mit einem Gegenanschlag 81 am Gewicht 78 zusammenwirkt. Die Spindel 79 ist mit einem Stellmotor 173 verbunden, wobei die ganze Anordnung ähnlich derjenigen ist, wie sie in anderem Zusammenhange bereits in der DE-A-38 39 778 für eine Gewichtsverstellung beschrieben worden ist.

Eine der Fig. 4b ähnliche Konstruktion könnte aber auch noch für einen anderen Zweck eingesetzt werden. Wenn nämlich beispielsweise eine nach unten führende und durch einen Schieber 67 (Fig. 4 a) abdeckbare Offnung vorgesehen ist, wie dies etwa auch aus Fig. 1 hervorgeht, so könnte eine (feder- oder) gewichtsbelastete Klappe 75 an einer Offnung 74 zusätzlich vorgesehen sein, um im Falle eines plötzlich auftretenden Staues innerhalb des Gehäuses 11 als Entlastungsklappe zu wirken. In diesem Falle könnte aber wohl eine Regelung der Belastung dieser Klappe entfallen.

Während im Falle der Fig. 4 b) das Kompaktierwerk 11 zusätzlich zu den Paddeln 12 auch vom Gehäuse 13 abstehende Statorwerkzeuge 82 aufweist, die eine Homogenisierung durch das Kämmen mit den Paddeln 12 fördern, fehlen solche Werkzeuge im Falle der Fig. 4 c). Hier ist eine grössere Klappe 175 an einem Hebel 83 befestigt, der mit einer Verstellwelle 176 verbunden ist. An der Verstellwelle 176 sitzt ein, lediglich strichliert angedeutetes Zahnrad 84, mit dem eine Zahnstange 85 in Eingriff steht. Diese Zahnstange 85 wird von einem Zylinderaggregat 86 betätigt, das über eine entsprechende Stellsteuerung 366 angesteuert wird, wobei letztere ihre Regelsignale von der Regeleinrichtung 64 erhält.

Es versteht sich, dass die Verstellung eines Auslauf-Verschlussorganes 67 bzw. 75 oder 175 auch zusätzlich zur Verstellung der Drehzahl des das Kompaktierwerk 11 antreibenden Motors 10 (Fig. 1) erfolgen kann. Beispielsweise geschieht dies nach Art einer Kaskadenregelung, indem erst die eine Regelung (z.B. die des Motors 10) betätigt wird und bei Erreichen der Grenzen des Regelbereiches die jeweils andere (z.B. die des Verschlussorganes). Es kann aber ein rascheres Ansprechen der Regelung und damit eine kürzere Regelzeitkonstante durch gleichzeitiges Ansprechen beider Regelungen erzielt werden.

Es sei bemerkt, dass die Beeinflussung der Kompaktierung auch dadurch vorgenommen werden kann, dass die Speisung des Mahlwerkes entsprechend reguliert wird, wie in Fig. 1 an Hand der strichpunktierten Linie 66 (Ausgang der Regelstufe 66) angedeutet ist. Dies bedeutet aber eine grössere Regelzeitkonstante und wird daher im allgemeinen nicht bevorzugt sein. Eine Ausnahme könnte allerding die Einbindung dieser Regelungsart in die oben besprochene Kaskadenregelung bilden.

Die Erfindung ist keineswegs auf die Messung relativ spröder Partikel, gegebenenfalls mit nachfolgender Regelung eines Mahlspaltes und/oder Mahldruckes, beschränkt, vielmehr können auch relativ formbare Partikel auf diese Weise gemessen werden. Ein Beispiel mag die Herstellung von Flocken (z.B. Haferflocken, Sojaflocken usw.) auf einem Quetschwalzwerk sein. Durch anschliessende Messung der Partikelgrösse und/oder (bevorzugt) des Schüttgewichtes lassen sich Rückschlüsse auf die durchschnittliche Flockengrösse ziehen, und daraus ermitteln, ob die erhaltene Flockengrösse innerhalb eines gewünschten Bereiches liegt. Davon lassen sich wiederum entsprechende Regeleingriffe zur Veränderung des Druckes der Walzen bzw. ihres Mahlspaltes ableiten.

Im folgenden wird nun die Messung der Teilchengrösse im Detail beschrieben. Aus der Literatur sind im Prinzip zwei verschiedene Geräte bekannt, mit welchen die spezifische Oberfläche der Schüttung gemessen werden kann.

Das sog. Blaine-Prinzip (DIN 66127) benützt eine Methode der Durchströmung der Schüttung mit zeitlich veränderlichem Druckabfall und das Lea- und Nurse-Gerät mit zeitlich konstantem Druckabfall. Beide Messprinzipien dienen zur Bestimmung der spezifischen Oberfläche der Schüttung, und der Erfindung liegt die Erkenntnis zugrunde, dass die spezifische Oberfläche eine Funktion der Teilchengrösse, insbesondere der mittleren, ist.

Eine Messung eines die spezifische Oberfläche bestimmenden Parameters und sein Vergleich mit entsprechenden Eichwerten ergibt daher die Kenntnis der Partikelgrösse.

Im Interesse einer automatisierten fliegenden Messung der Teilchengrösse zum Zweck der Regelung eines Mahlwerks ist es selbstverständlich nicht wünschenswert, von Zeit zu Zeit eine Probeschüttung zu entnehmen und diese einer manuellen Messung in einem der oben erwähnten Geräte zuzuführen, die Messung auszuwerten und eine entsprechende Regelung des Mahlwerks händisch vorzunehmen, sondern all diese Vorgänge sollen automatisch durchführbar sein.

Da die Messung der spezifischen Oberfläche einer Schüttung in beiden genannten Geräten eine genau definierte Schüttungsgeometrie vorschreibt, welche bei automatisch ablaufenden Probenherstellungen nur in bestimmten Grenzen erreicht werden kann, kann es notwendig sein, Korrekturmöglichkeiten vorzusehen, welche die Abweichung der reellen Probengeometrie von der vorgeschriebenen Geometrie zu kompensieren gestatten.

Beispielsweise ist beim Blaine-Prinzip bei vorgegebenem Probenquerschnitt und Höhe die Probe mit einer bestimmten definierten Kraft zu verdichten, um eine die Teilchengrösse repräsentierende Porosität zu erreichen. Bei der Verdichtung mittels eines Kolbens im Messzylinder unter einer definierten Kraft wird jedoch eine Probenhöhe erreicht, welche nicht konstant ist, sondern von der Teilchengrösse selbst, aber auch von anderen Parametern wie Luftfeuchtigkeit u.dgl. abhängt, soferne nicht die Probenmenge zum Zwecke der Konstanthaltung des Volumens verändert wird, wie dies nach Blaine vorgesehen ist.

Es muss daher vorgesehen werden, dass die reelle Probenhöhe bestimmbar ist, damit zusammen mit dem Probengewicht die Porosität errechnet werden kann, welche in die Bestimmungsgleichung der spezifischen Oberfläche

$$Sv = \frac{k}{(1 - epsilon)} \cdot Wurzel \ \frac{epsilon^3 \cdot t}{eta}$$

eingeht. Hierbei bedeuten Sv die spezifische Oberfläche pro Volumenseinheit, epsilon die Porosität, eta die Viskosität der Luft und t die Durchströmungsdauer eines definierten Luftvolumens durch die Schüttung. k ist ein, der spezifischen Systemgeometrie entsprechender Proportionalitätsfaktor.

Bei Durchführung einer Durchströmungsmessung wird z.B. t, die Dauer der Durchströmung der Probe von einem bestimmten Luftvolumen ermittelt und über die obige Gleichung die spezifische Oberfläche, und daraus die Partikelgrösse in einem Mikroprozessor berechnet. Hierzu kann eine formelmässige Berechnung von Sv und/ oder eine Bestimmung der Partikelgrösse durch Vergleich der spezifischen Oberfläche mit einer Eichkurve erfolgen. Die Bestimmung der Eichkurve erfolgt mittels identisch ablaufenden Durchströmungsmessungen von Proben, deren mittlere Partikelgrössen bekannt sind, und die unter Aufwendung der gleichen Kraft verdichtet worden sind und eine definierte Probenhöhe aufweisen.

Wie weiter oben erwähnt wurde, muss für die Kompensierung der Abweichung der reellen Probenhöhe von der vorgeschriebenen Höhe, allenfalls Sorge getragen werden. Dies kann nach Kenntnisnahme der reellen Probenhöhe auf mathematischem Wege direkt während der Berechnung im Mikroprozessor erfolgen, wobei die Grösse des Proportionalitätsfaktors k entsprechend korrigiert wird. Die reelle Probenhöhe kann beispielsweise über den Weg des Verdichtungskolbens gemessen werden, wobei die Abweichung der reellen Endstellung des Verdichtungskolbens von der angestrebten Endstellung als Korrekturwert benützt werden kann. Die Bestimmung der reellen Endstellung des Verdichtungszylinders kann durch Wegmessung oder Positionsmessung evtl. mechanischer, elektrischer oder optischer Weise erfolgen. Selbstverständlich ist es auch möglich, den Füllstand im Probenzylinder nach Verdichtung direkt zu messen. In der Praxis wird man eine Variante wählen, die bezüglich Genauigkeit, Geschwindigkeit und Kosten den übrigen Bedingungen angepasst ist.

Die Fig. 1 bis 3 zeigen, wie oben erwähnt, eine erfindungsgemässe Messeinrichtung, welche fähig ist, in gewünschten Zeitabständen Probenzylinder zu füllen, zu komprimieren und einer Messstation zuzuführen, in der ein bestimmtes Luftvolumen durch die Probe geschickt wird.

Die oben angegebene Gleichung gilt für eine Messung nach dem Blaine-Verfahren mit variablem Druck und ist hier als Beispiel zu verstehen. Selbstverständlich können die Durchströmungsmessungen auch auf andere Art und Weise durchgeführt werden, beispielsweise mit konstantem Druckabfall, wobei die Luftmenge in der Zeiteinheit bestimmt wird oder der Druckabfall bei erzwungener Durchströmung eines definierten Gasvolumens in bestimmter Zeit gemessen wird, wie nach dem Prinzip von Lea- und Nurse.

Wie weiter oben erwähnt, kann diese Eichkurve gegebenenfalls durch eine einzelne Referenzmessung gewonnen werden, da es sich herausgestellt hat, dass der Zusammenhang von Strömungswiderstand und Partikelgrösse in weiten Bereichen linear und proportional ist. Dies wird an Hand der nachfolgenden Tabelle deutlich, die die Messungen an Hand von vier Proben T12 bis T15 veranschaulicht.

Diese Proben wurden auf Grund der Messung des Druckabfalles mit einem Gerät nach Fig. 3 auf Grund verschiedener Vorverdichtungen durch den Kolben 37 berechnet und ihre tatsächliche, auf herkömmlichem Wege bestimmte Partikelgrösse damit verglichen. Fig. 5 zeigt, dass sich dabei eine lineare Korrelation zwischen dem Druckabfall x und der Partikelgrösse für $y_{x50}$ und $y_{x90}$ ergibt, wobei es sich bei $y_{x50}$ um einen der durchschnittlichen Mahlfeinheit entsprechenden Wert handelt, bei $y_{x90}$ praktisch um einen der Maximalpartikelgrösse entsprechenden Wert. Die Tabelle veranschaulicht sehr gut, dass der Korrelationskoëffizient R eine weitgehende Entsprechung mit Werten zwischen -0,989 und -0,997 angibt. Dabei wurde der Ordnung halber auch der Korrelationskoëffizient R (L) für den Zusammenhang zwischen dem Mahlspalt und den durchschnittlichen (x50) und maximalen (x90) Partikelgrössen berechnet und findet sich in der Grössenordnung von 0,997 bis 0,999.

Die Rückrechnung auf die Mahlfeinheit erfolgt nach einer linearen Regressionsrechnung

$$y = m \cdot x + b \ ,$$

wobei

y   der gesuchte Wert entweder für jene Partikelgrösse ist, die der durchschnittlichen Mahlfeinheit entspricht ($y_{x50}$) oder annähernd der maximalen Partikelgrösse ($y_{x90}$) ;

m   ist ein dem Anstiegswinkel der Geraden für $y_{x50}$ bzw. $y_{x90}$ in Fig. 5 entsprechender Wert;

x   der Druckabfall in mbar; und

b   bedeutet den Wert am Schnittpunkt der Geraden $y_{x50}$ bzw. $y_{x90}$ der Fig. 5 mit der y-Achse, d.h. die Entfernung dieses Schnittpunktes vom Nullpunkt.

Man sieht, dass der Anstiegswinkel, d.h. der Wert von m je nach der gesuchten Partikelgrösse ($y_{x50}$ oder $y_{x90}$), unter der Voraussetzung gleichen Gasdruckes durch die Probe im Probengefäss 24, verschieden ist. Dagegen hängt b im wesentlichen vom Produkt ab, insoferne bei gleicher Steigung (Wert m) der Abstand der Korrelationsgeraden von der y-Achse für den Schnittpunkt mit dieser letzteren massgebend ist. Selbstverständlich liesse sich die Berechnung auch auf andere Weise durchführen, doch erscheint die oben beschriebene als die für praktische Zwecke günstigste.

Grundsätzlich liessen sich auch nicht-lineare Regressionsrechnungen anwenden, um zum gewünschten Ergebnis zu kommen, doch ist der Zusammenhang, wie Fig. 5 deutlich zeigt, so weitgehend linear, dass mit der obigen Berechnungsmethode das Ziel auf vereinfachtem Wege erreicht wird, was sich letztlich auch in einer Vereinfachung des Rechners 45 auswirkt.

Die Partikelgrössenverteilung der vier Proben T12 bis T15 ist der Fig. 6 zu entnehmen, in der an der vertikalen Achse praktisch die Prozentwerte aufgetragen sind, auf der x-Achse die Partikelgrösse in micron, so dass sich bei 50% die durchschnittliche Feinheit (gemessen mit einer herkömmlichen Methode) feststellen lässt, bei 90% etwa (nicht ganz) die maximale Partikelgrösse.

Tabelle  Uebersicht der Messergebnisse und Werte der linearen Regressionsrechnung  $y = m * x + b$
R = Korrelationskoeffizient

| Probe Nr. | Mahlspalt L (mm) | Partikelgrösse (micron) | | Druckabfall p (mbar) bei verschiedenen Vorverdichtungen | | |
| --- | --- | --- | --- | --- | --- | --- |
| | | x50 | x90 | F = 0 N | F = 250 N | F = 500 N |
| T 12 | 0.20 | 333 | 600 | 51 | 128 | 159 |
| T 13 | 0.24 | 403 | 690 | 41 | 96 | 118 |
| T 14 | 0.27 | 472 | 766 | 29 | 65 | 80 |
| T 15 | 0.30 | 546 | 845 | 21 | 48 | 55 |
| R (x90) | | | | -0.997 | -0.993 | -0.996 |
| R (x50) | | | | -0.996 | -0.989 | -0.993 |
| R (L) | | 0.997 | 0.999 | | | |

EP 0 485 772 B1

**Patentansprüche**

1. Mahlwerk mit mindestens zwei miteinander einen Mahlspalt begrenzenden Walze (2) und einer eine Stelleinrichtung (48, 49) zur Einstellung mindestens eines den Mahlspalt bestimmenden Parameters umfassenden Regeleinrichtung (47), dadurch gekennzeichnet, dass an den Ausgang des Mahlwerkes eine Messeinrichtung angeschlossen ist, die eine Einrichtung (16, 21) zur, insbesondere diskontinuierlichen, Entnahme von Schüttgutproben eines kontinuierlichen Schüttgutflusses am Ausgange des Mahlwerks, eine Manipulationseinrichtung (28) zum Transport eines zur Füllung mit Schüttgut dienenden Probengefässes (24), zu einer Mehrzahl von Stationen (24', 24"), wovon eine Station eine Fullstätion und eine andere Station eine Messstation (24') ist, sowie eine, die in der Messstation ermittelbaren Werte in vorgegebener Weise zu einem Resultat verknüpfenden Recheneinrichtung (45) zur Errechnung der Mahlfeinheit aufweist und dass die Ausgangssignale der Regeleinrichtung (45) der Regeleinrichtung (47) zum Vergleich mit einem Sollwert übermittelbar sind, an welche Regeleinrichtung (47) die genannte Stelleinrichtung (48, 49) angeschlossen ist.

2. Vorrichtung nach Anspruch 1, dadurch gekennzeichnet, dass eine der Stationen mit einer, einen Kolben aufweisenden Verdichtungseinrichtung ausgestattet ist, mittels welcher die Schüttgutprobe verdichtet wird.

3. Vorrichtung nach Anspruch 1 oder 2, dadurch gekennzeichnet, dass die Messstation (24') eine. einer Gasdurchströmungsmessung dienende Gasströmungseinrichtung (40, 41) im wesentlichen luftdicht an das, an seiner Unterseite offene Probengefäss anschliessbar ist.

4. Vorrichtung nach einem der vorhergehenden Ansprüche, dadurch gekennzeichnet, dass die Manipulationseinrichtung (28) eine Verschiebeeinrichtung (28) für das Probengefäss (24) sowie eine Bodenplatte (26) aufweist, auf welcher das unten offene Probengefäss (24) mittels des Verschiebekolbens (28) von einer der Stationen (24') zur anderen verschiebbar ist.

5. Vorrichtung nach einem der vorhergehenden Ansprüche, dadurch gekennzeichnet, dass eine weitere Station (24") mit einer Wiegeeinrichtung (55-61) vorgesehen ist.

6. Vorrichtung nach einem der vorhergehenden Ansprüche, dadurch gekennzeichnet, dass sie ein über ein Permeabilitätsmessgerät (29), die Recheneinrichtung (45) und die Regeleinrichtung (47) verstellbares Stellglied (48, 49) aufweist, durch das der Walzenabstand veränderbar ist, wobei orzugsweise dem Stellglied (48, 49) ein Inkrementalantrieb zugeordnet ist, und dass vorzugsweise zur Bestimmung einer definierten Ausgangslage die Walzen (2) zu Beginn des Betriebes zunächst in eine vorbestimmte Offenstellung und erst dann in die jeweils gewunschte, den Mahlspalt definierende Position bringbar sind.

7. Vorrichtung nach einem der vorhergehenden Ansprüche, dadurch gekennzeichnet, dass sie ein Kompaktierorgan (11) am Ausgange des Mahlwerkes (1) aufweist, wobei an diesem Kompaktierorgan (11) zur Veränderung der Schüttdichte die Antriebsgeschwindigkeit bzw. der Kompaktierdruck und/oder der Auslassquerschnitt veränderbar ist,

8. Vorrichtung nach Anspruch 7, dadurch gekennzeichnet, dass zur Veränderung des Auslassquerschnittes ein Verschlussorgan (67; 75; 175), wie eine Auslassklappe (75; 175), ein Auslassschieber (67) vorgesehen ist, und dass die Stelleinrichtung einen Antrieb (73; 173; 86) für die Verstellung dieses Verschlussorganes (67; 75; 175) aufweist.

**Claims**

1. A grinding gear comprising at least two rollers (2) together delimiting a grinding gap, and a control unit (47) comprising a control device (48, 49) for setting at least one parameter determining the grinding gap, characterised in that measuring equipment is connected to the exit of the grinding gear which said measuring equipment comprises a device (16, 21) for obtaining, in particular obtaining in a discontinuous way, samples of bulk material, of a continuous flow of bulk material at the exit of the grinding gear, a manipulation device (28) for transporting a sample container (24) to be filled with bulk material to a plural number of stations (24', 24") with one station being a filling station and another station being a measuring station (24'), as well as a computer (45) which in a pre-set way integrates to a result the values which can be determined in the measuring station, for the purpose of calculating the grinding fineness, and that the output signals of the computer (45) can be transmitted to the control unit (47) for comparison with a desired value, with the said control device (48, 49) being connected to the control unit (47).

2. A device according to claim 1, characterised in that one of the stations is equipped with a compacting device comprising a piston, by means of which compacting device the sample of bulk material is compacted.

3. A device according to claim 1 or 2, characterised in that the measuring station (24') of gas-flow equipment (40, 41) used to carry out gas-flow measurement, is connectable in an essentially airtight way to the sample container open at the bottom.

4. A device according to one of the preceding claims, characterised in that the manipulation device (28) comprises a sliding device (28) for the sample container (24) as well as a floor plate (26) on which the sample container (24) which is open at the bottom is slideable from one of the stations (24') to the other by means of the sliding piston (28).

5. A device according to one of the preceding claims, characterised in that a further station (24") with a weighing device (55-61) is provided.

6. A device according to one of the preceding claims, characterised in that a control element (48, 49) which by way of a permeability measuring instrument (29) is able to adjust the computer (45) and the control unit (47), by which control element (48, 49) the grinding gap can be changed, with preferably an incremental drive being allocated to the control element (48, 49), and in that preferably, to determine a defined initial position, the rollers (2) at the start of operations are first able to be positioned in a predetermined open position and only later able to be positioned in the respective desired position defining the grinding gap.

7. A device according to one of the preceding claims, characterised in that it comprises a compacting device (11) at the exit of the grinding gear (1), with the drive speed or the compacting pressure and/or the outlet diameter being able to be changed at this compacting device (11) so as to change the bulk density.

8. A device according to claim 7, characterised in that for changing the outlet diameter, a closure unit (67, 75, 175) such as an outlet flap (75, 175), an outlet slide (67) is provided, and that the control device comprises a drive (73, 173, 86) for adjusting this closure unit (67, 75, 175).

**Revendications**

1. Broyeur comprenant au moins deux cylindres (2) délimitant entre eux un intervalle de broyage, et un dispositif de réglage (47) comportant un dispositif d'ajustement (48, 49) permettant l'ajustement d'au moins un paramètre définissant l'intervalle de broyage, caractérisé en ce qu'on raccorde à la sortie du broyeur un dispositif de mesure comprenant un dispositif (16, 21) pour le prélèvement, en particulier discontinu, d'échantillons de produit en vrac pris dans un flux continu de produit en vrac à la sortie du broyeur, un dispositif de manipulation (28) permettant l'acheminement d'un récipient à échantillons (24) destiné à être rempli de produit en vrac, vers une multiplicité de postes (24', 24"), postes dont l'un est un poste de remplissage et l'autre un poste de mesure (24'), ainsi qu'un dispositif de calcul (45) servant à calculer la finesse et associant de manière définie au préalable les valeurs relevées dans le poste de mesure à un résultat, et en ce que les signaux de sortie du dispositif de calcul (45) peuvent être transmis au dispositif de réglage (47) pour comparaison à une valeur de consigne, dispositif de réglage (47) auquel est raccordé le dispositif d'ajustement (48, 49) précité.

2. Dispositif selon la revendication 1, caractérisé en ce que l'un des postes est équipé d'un dispositif de compactage présentant un piston et au moyen duquel l'échantillon de produit en vrac est densifié.

3. Dispositif selon la revendication 1 ou 2, caractérisé en ce que le poste de mesure (24') présente un dispositif d'écoulement de gaz (40, 41) servant à la mesure de l'écoulement du gaz, lequel peut être raccordé de manière sensiblement hermétique au récipient à échantillons ouvert sur sa face inférieure.

4. Dispositif selon l'une des revendications qui précèdent, caractérisé en ce que le dispositif de manipulation (28) présente un dispositif de déplacement (28) pour le récipient à échantillons (24), ainsi qu'une plaque de base (26) sur laquelle le récipient à échantillons (24) ouvert en bas peut être glissé d'un poste (24') à l'autre au moyen du piston de déplacement (28).

5. Dispositif selon l'une des revendications qui précèdent, caractérisé en ce qu'un autre poste (24") comprenant un dispositif de pesage (55-61) est prévu.

6. Dispositif selon l'une des revendications qui précèdent, caractérisé en ce qu'il présente un vérin (48, 49) ajustable par l'intermédiaire d'un appareil de mesure de perméabilité (29), du dispositif de calcul (45) et du dispositif de réglage (47), et par lequel l'entre-deux cylindres peut être varié, une commande à pas de progression étant de préférence associée au vérin (48, 49), et en ce que, de préférence, pour déterminer une position de sortie définie, les cylindres (2) peuvent être amenés dans une première phase, au début du fonctionnement, dans une position d'ouverture déterminée au préalable, et puis seulement dans la position souhaitée suivant le cas, définissant l'intervalle de broyage.

7. Dispositif selon l'une des revendications qui précèdent, caractérisé en ce qu'il présente un organe de compactage (11) à la sortie du broyeur (1), la vitesse d'entraînement, voire la pression de compactage et/ou la section de sortie pouvant être modifiée au niveau de cet organe de compactage, pour faire varier la densité en vrac.

8. Dispositif selon la revendication 7, caractérisé en ce que pour faire varier la section de sortie, on a prévu un organe d'obturation (67; 75; 175) tel qu'un clapet de sortie (75; 175), une vanne d'évacuation (67), et en ce que le dispositif d'ajustement présente une commande (73; 173; 86) pour le déplacement de cet organe d'obturation (67; 75; 175).

Fig.1

Fig. 2

Fig.3

Fig. 4 a)

b)

c)

Fig. 5

Fig.6